# NEUE EUROPÄISCHE PATENTSCHRIFT

(11) **EP 0 436 159 B2**
(45) Veröffentlichungstag und Bekanntmachung des Hinweises auf die Entscheidung über den Einspruch: **31.01.2001**
(45) Hinweis auf die Patenterteilung: 05.04.1995
(21) Anmeldenummer: 90123891.5
(22) Anmeldetag: 12.12.1990
(51) Int. Cl.: C09J 7/02, C09J 133/06, A61L 15/16

(54) **Schmelzhaftkleber auf Acrylatbasis**
Hot melt pressure sensitive adhesive based on acrylates
Adhésive sensible à la pression thermofusible à base acrylique

(30) Priorität: 21.12.1989 DE 3942232
(43) Veröffentlichungstag der Anmeldung: 10.07.1991
(73) Patentinhaber: Beiersdorf Aktiengesellschaft, 20245 Hamburg (DE)
(72) Erfinder: Massow, Klaus, W-2000 Hamburg 65 (DE); Karmann, Werner, Dr., W-2000 Hamburg 73 (DE); Kiessling, Günther, Dr., W-2000 Hamburg 61 (DE)

(56) Entgegenhaltungen:
- EP-A- 0 024 839
- EP-A- 0 346 734
- FR-A- 1 407 951
- GB-A- 2 048 274
- US-A- 3 725 115

## Beschreibung

Gegenstand der Erfindung ist ein Verfahren zur Herstellung von Schmelzhaftklebern auf Acrylatbasis durch Bestrahlung auf einem Substrat.

Mit Copolymerisaten aus Acrylsäureestern lassen sich bekanntlich druckempfindliche Klebemassen herstellen, die hohe Anforderungen der Anwender vielfach erfüllen können. Zu den besonderen Vorteilen von Acrylatklebemassen gehört das ausgewogene Verhältnis zwischen adhäsivem und kohäsivem Verhalten, d.h., trotz eines starken Widerstandes gegen Scherbeanspruchung läßt sich dennoch eine hohe Ablöseadhäsion und gute Anfaßklebrigkeit erzielen.

Hinzu kommt, daß Polyacrylate sich gegenüber vielen Umwelteinflüssen, wie z.B. UV-Strahlung oder Wärme- und Feuchtigkeitseinwirkung, günstig verhalten. Dies gilt im besonderen Maße für Klebemassen, die nach dem Verfahren der Lösungsmittelpolymerisation hergestellt wurden, da hierbei auf feuchtigkeitsempfindliche Detergentien, wie sie im allgemeinen bei einer Emulsionspolymerisation verwendet werden, verzichtet werden kann.

Das besonders in jüngster Zeit steigende Kosten- und Umweltbewußtsein ließ den Wunsch aufkommen, selbstklebende Artikel zu produzieren, bei deren Herstellung keine Lösungsmittel mehr anfallen. Hierfür ist das Schmelzauftragsverfahren geeignet, bei dem die Klebemassen im geschmolzenen Zustand auf ein Substrat aufgebracht werden.

Die Schwierigkeit bei der Formulierung von Schmelzhaftklebern besteht darin, daß sie einerseits schon bei nicht allzu hohen Temperaturen fließfähig sein müssen. Bei zu starker Temperaturbelastung besteht sonst die Gefahr einer Zerstörung des Klebers durch Depolymerisation oder durch Vergelung.

Andererseits muß die Klebemasse nach der Beschichtung eine möglichst hohe Kohäsion aufweisen, so daß es nicht zur Rückstandsbildung beim Ablösen der Klebestreifen vom Untergrund kommt, bzw. daß auch Scherfestigkeiten erzielt werden können, die selbst bei erhöhten Temperaturen technische Anwendungen gestatten. Diese sich widersprechenden Forderungen schienen lange Zeit kaum vereinbar zu sein, so daß immer nur einer Eigenschaftsrichtung der besondere Vorzug gegeben werden mußte.

So wurden z.B. im US-Patent 4,456,741 unvernetzte Schmelzhaftkleber vorgestellt, in denen die Acrylsäureester-Komponenten mit Styrol und N-Vinylpyrrolidon modifiziert wurden. Diese beiden letzten Monomere verleihen dem Polymerisat eine höhere Glasübergangstemperatur, und damit eine bessere Kohäsivität bei Umgebungstemperatur. Ihr Einsatz in Klebemassen geht aber zu Lasten der Selbstklebeeigenschaften Tack und Klebkraft, besonders bei tiefen Temperaturen, und verschlechtern das Schmelzverhalten.

Ein anderer Weg, um zu schmelzfähigen Systemen zu kommen, ist die reversible Vernetzung, bei der intermolekulare Bindungen über Nebenvalenzkräfte gebildet werden. Hier sei das US-Patent 4,762,888 genannt, dem zufolge die Vernetzung von Polyurethanacrylaten unterschiedlichen Molekulargewichts über Wasserstoffbrückenbindungen erfolgt.

Frühere Vorschläge, wie im US-Patent 3,925,282 beschrieben, sahen eine Vernetzung von tertiäre Amine enthaltenden Acrylat-Schmelzhaftklebern mit Salzen aus organischen Säuren und Übergangsmetallen vor. Diese Bindungen brechen in der Wärme auf, so daß auf diese Weise die Fließfähigkeit gewährleistet sein soll.

Darüber hinaus wurde auch versucht, wie z.B. in der US-Patentschrift 4,656,213 oder im EP 259 968 beschrieben, die Kohäsivität über Polystyrol-Domänen zu erreichen: Bei dieser Art von Vernetzung werden die üblicherweise eingesetzten Acrylsäureester mit Makromonomeren auf Polystyrol-Basis mischpolymerisiert, so daß Acrylpolymere mit aufgefropften Polystyrolketten entstehen. Da diese sich aufgrund thermodynamischer Unverträglichkeit mit den Acrylatteilen zu sogenannten Domänen zusammenlagern, kommt es zu einer physikalischen Vernetzung.

Es ist aber leicht einzusehen, welchen wesentlichen Nachteil eine reversible Vernetzung in jedem Fall hat: Es lassen sich auf diese Weise keine wärmescherfesten Klebemassen herstellen, wie sie aber durch die gestiegenen Anforderungen z.B. im Fahrzeug- oder Flugzeugbau vermehrt verarbeitet werden.

Ein effektiver Weg, um zu Klebemassen mit hohen Kohäsionsfestigkeiten zu gelangen, ist die Verarbeitung thermoplastischer Systeme, bei denen nach beendeter Schmelzbeschichtung mittels energiereicher Strahlung irreversible, intermolekulare chemische Bindungen geknüpft werden, die dreidimensionale Netzwerke ergeben. Als Beispiele für diese Vorgehensweise seien das US-Patent 3,725,115 genannt, in dem Copolymerisate aus Acrylatestern, Vinylacetat und geringen Anteilen Acrylsäure und Diacetonacrylamid mittels energiereicher Elektronenstrahlen gehärtet wurden.

Des weiteren wurde, wie in der DE-A-3 015 463 beschrieben, versucht, die Vernetzbarkeit der Acrylatschmelzhaftkleber durch Einbau von Doppelbindungen zu erhöhen: Hierzu wurde Allylacrylat beziehungsweise Allylmethacrylat über die acrylische Doppelbindung mit Acrylsäureestern wie 2-Ethylhexylacrylat mischpolymerisiert, während die Allylgruppierung die Strahlenausbeute erhöhen sollte.

In ähnlicher Weise wurde auch gemäß US-Patent 4,438,177 vorgegangen: Hierin wurde die Herstellung von niedermolekularen, flüssigen Polymeren mit K-Werten zwischen 10 und 30 beschrieben, die mit Hilfe von Vernetzern wie Allyl(meth)acrylat oder polyfunktionellen (Meth)acrylaten gehärtet wurden. Besonders letztere sind thermisch aber instabil und neigen bei erhöhten Temperaturen zur Vergelung, was ihre Einsetzbarkeit stark einschränkt.

In der FR-A-1 407 951 sind bereits unvernetzte TBA enthaltende Acrylatcopolymer-Haftkleber beschrieben, die für den Auftrag aus Lösungsmitteln bestimmt sind.

Weiterhin sind aus EP-A-0 346 734 UV-vernetzbare Massen auf Basis von (Meth)acrylester-Copolymerisaten bekannt.

In allen vorstehend beschriebenen Fällen war aber die erreichbare Wärmescherfestigkeit gering. Die Scherstandzeiten bei 80°C lagen in keinem Fall höher als im Bereich von nur wenigen hundert Minuten. In der nachfolgenden detaillierten Beschreibung der neuen, erfindungsgemäßen Acrylatschmelzhaftkleber werden Klebmassen formuliert, die nach Bestrahlung mit relativ geringen Dosen unter ähnlichen Bedingungen mehrfach längere Scherstandzeiten aufweisen. Ferner haben mit Diacetonacrylamid erhaltene Copolymerisate den Nachteil einer nicht immer befriedigenden thermischen Stabilität.

Aufgabe der Erfindung ist die Herstellung von Schmelzhaftklebern auf Acrylatbasis, das heißt druckempfindlichen Klebemassen oder Acrylatklebern, die sich gut aus der Schmelze verarbeiten und sich mit relativ geringen Dosen stahlenvernetzen lassen und danach über ein ausgewogenes Haftklebeverhalten mit sowohl gutem Adhäsionsvermögen, wie auch gleichzeitig hoher Kohäsion verfügen und sich durch eine gute Scherfestigkeit auszeichnen.

Weiterhin sollen sich die Klebmassen nicht nur für Verklebungen allgemein, z.B. im Bereich der Technik eignen, sondern auch im medizinischen Bereich Verwendung finden, indem sie beispielsweise auf der Haut, also in feuchter Umgebung, dauerhaft kleben, ohne zu Hautirritationen zu führen.

Diese Aufgabe wird gelöst durch ein Verfahren zur Herstellung von Schmelzhaftklebern auf Acrylatbasis durch Bestrahlung auf einem Substrat, dadurch gekennzeichnet, daß man
a) 55 bis 98 Gew.-% mindestens eines acrylischen Monomers mit 2 bis 30 Gew.-% N-tert.-Butylacrylamid (TBA) copolymerisiert, wobei
b) die zu polymerisierende Reaktionsmischung (von a) zusätzlich eine oder mehrere ethylenisch ungesättigte Verbindungen enthalten kann, die nicht der Gruppe der acrylischen Monomere von a) angehören und wobei die zu polymerisierende Reaktionsmischung (von a) keine UV-vernetzbare Massen auf Basis von (Meth)acrylester-Copolymerisaten eines K-Werts von 15 bis 100, mit einem Gehalt von 0.01 bis 10 Gew.%, bezogen auf die Copolymerisate an einpolymerisierten Monomeren der allgemeinen Formel I 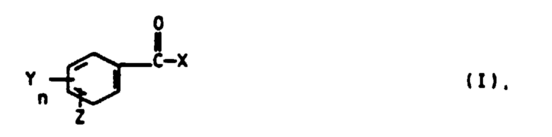 in der
   X für eine 1 bis 3 C-Atome enthaltende Alkylgruppe oder einen gegebenenfalls durch n-Gruppen Y substituierten Phenylrest,
   Y für -H, -CF₃, -O-Alkyl- und/oder Alkyl-COO- mit jeweils 1 bis 4 C-Atomen in der Alkylgruppe, Halogen, -CN, -COOH oder eine nicht ortho-ständige -OH-Gruppe,
   n für 0 bis 4 und
   Z für eine Gruppe der allgemeinen Formel 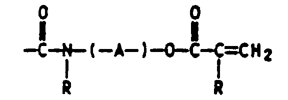 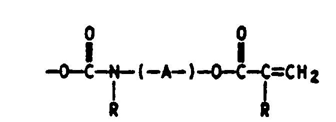 oder 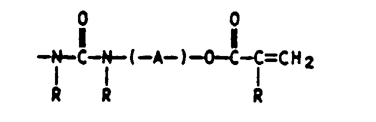 steht, in der
   R H oder C₁- bis C₄-Alkyl und
   A einen 2 bis 12 C-Atome aufweisenden Alkylen-, Oxaalkylen-oder Polyoxaalkylenrest bedeuten,
   enthält,
c) das erhaltene feste Polymerisat erwärmt und in fließfähiger oder flüssiger Form auf ein Substrat aufbringt, wobei die Massen der Schritte a) bis c) gegebenenfalls Zusatz- und Hilfsstoffe enthalten können, und
d) das beschichtete Substrat mit energiereicher und ionisierender Strahlung bestrahlt.

Erfindungsgemäße acrylische Monomere a) sind vorzugsweise Alkylester der Acrylsäure oder Methacrylsäure der allgemeinen Formel I 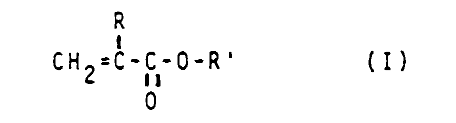 in der R Wasserstoff oder die Methylgruppe und R' ein geradkettiger oder verzweigter Alkylrest mit 1 bis 18 C-Atomen oder ein Cycloalkylrest mit 3 bis 18 C-Atomen ist. Wird nur ein acrylisches Monomer verwendet, besitzt dessen Alkylrest vorzugsweise eine Anzahl von 4 bis 8 C-Atomen. Gemische können mehrere, beispielsweise 2 bis 5 verschiedene Monomere a) enthalten. Der Anteil der acrylischen Alkylester a) beträgt 55 bis 98 Gew.-% bezogen auf die Gesamtmenge an Monomeren in der Reaktionsmischung, bevorzugt 70 bis 95 Gew.-%, insbesondere 70 bis 90 Gew.-%.

Für die Verwendung im medizinischen Bereich, beispielsweise als Pflasterklebmasse, enthalten die Monomergemische vorzugsweise 80 bis 95 Gew.-%, insbesondere 90 bis 95 Gew.-% acrylische Monomere.

Als Monomere aus dieser Gruppe eignen sich beispielsweise n-Butyl- und Isobutylacrylat und -methacrylat, Amyl- und Isoamylacrylat, Hexyl- und Heptylacrylat, n-Octyl-, Iso-Octyl-, 2-Ethylhexyl-, Decyl- und iso-Decylacrylat bzw. -methacrylat aber auch Alkylester wie Lauryl- und Stearylacrylat bzw. -methacrylat oder cyclische Alkylester wie beispielsweise Cyclohexyl- oder Isobornylacrylat bzw. -methacrylat.

Der Anteil an N-tert.-Butylacrylamid (TBA) liegt bei 2 bis 30 Gew.-%, bezogen auf die Gesamtmenge an Monomeren in der Reaktionsmischung, insbesondere aber bei 5 bis 20 Gew.-%.

Für die Verwendung im medizinischen Bereich enthalten die Monomerengemische vorzugsweise 5 bis 20 Gew.-%, insbesondere 5 bis 10 Gew.-% TBA.

Besonders geeignet sind für diese medizinischen Zwecke Monomerengemische a) aus acrylischen Monomeren und TBA ohne einen Gehalt an ethylenisch ungesättigten Verbindungen b). Der Anteil an TBA beträgt vorzugsweise 5 bis 20 Gew.-%, insbesondere 5 bis 10 Gew.-% dieser Gemische.

Weiterhin können die erfindungsgemäßen Reaktionsmischungen gemäß a) wahlweise eine oder mehrere, insbesondere 2 oder 3, der genannten ungesättigten Verbindungen b) enthalten, die vorzugsweise eine oder mehrere, insbesondere zwei oder drei, polare Gruppen tragen. Geeignet sind solche Gemische für die allgemeine oder technische Verwendung oder aber, wenn besonders niedrige Auftragstemperaturen der Schmelze erforderlich sind.

Bevorzugte Monomere sind Vinylacetat, N-Vinylpyrrolidon, N-Vinylcaprolactam, Monoester und Diester der Maleinsäure und Fumarsäure wie zum Beispiel Maleinsäuremonoethylester, Maleinsäuremonobutylester, Maleinsäurediisopropylester, Fumarsäuredimethylester, Fumarsäuredibutylester oder Fumarsäuredioctylester.

Besonders bevorzugt werden Monomere, die polare Gruppen enthalten. Bevorzugte polare Gruppen sind Carboxylgruppen oder deren Anhydride, Carbonyl , Amino, substituiertes Amino, C₁₋₈-Mono- oder- Dialkylamino, Hydroxyl , C₁₋₆-Alkoxy, oder Halogen, z.B. Fluor, Chlor oder Brom. Von diesen polaren Monomeren sind insbesondere die Acrylsäure, Methacrylsäure, Crotonsäure oder Maleinsäure oder ihre Anhydride geeignet. Ganz besonders bevorzugt werden Maleinsäureanhydrid und Maleinsäure.

Darüber hinaus eignen sich auch Itaconsäure und Fumarsäure sowie ungesättigte Amide wie beispielsweise Acrylamid oder Methacrylamid.

Der Anteil dieser ungesättigten Verbindungen b) beträgt vorzugsweise 1 bis 15 Gew.-%, besser aber, um die Schmelzfähigkeit nicht zu beeinträchtigen, bevorzugt 1 bis 10 Gew.-% bezogen auf die Gesamtmenge an Monomeren, insbesondere aber 1 bis 5 Gew.-% und besonders bevorzugt 1 bis 3 Gew.-% oder 1 bis 2 Gew.-%.

Zusatz- und Hilfsstoffe wie Polymerisationsinitiatoren (z.B. 0,05 - 1%), Regler (z.B. 10 ppm - 10%), Antioxydantien (z.B. 0,1 - 1%), Sensibilisatoren für die UV-Vernetzung (z.B. 0,5 - 7%), Füllstoffe (z.B. 0,1 - 50%), Harze oder Harzsysteme (z.B. 1 - 40%) oder antimikrobiell wirkende Stoffe (z.B. 0,1 - 30%, vorzugsweise 1 - 10%), können gegebenenfalls, vorzugsweise in den genannten Gewichtsmengen, bezogen auf das Gesamtgewicht in den erfindungsgemäßen Klebemassen enthalten sein.

Die erfindungsgemäßen Schmelzhaftkleber können durch übliche Polymerisationsverfahren wie Suspensions-, Emulsions- oder Lösungsmittelpolymerisation hergestellt werden, wobei der Polymerisation mit Lösungsmitteln der Vorzug gegeben wird, da solche Klebmassen frei von Emulgatoren sind, was beispielsweise für eine Hautverklebung in Wundnähe von Vorteil ist. Zudem lassen sich organische Lösungsmittel nach beendeter Polymerisation im Vakuum mit geringem Energieaufwand vollständig aus dem Polymerisat entfernen und stehen nach Kondensation für weitere Polymerisationen erneut zur Verfügung. Das erhaltene, von Lösungsmitteln bzw. Wasser befreite Polymerisat wird als festes (bei Raumtemperatur) Polymerisat bezeichnet.

Die erfindungsgemäßen Klebemassen werden am einfachsten durch freie, radikalische Polymerisation hergestellt. Diese Technik macht eine besondere Aufbereitung von Monomeren und Lösungsmitteln entbehrlich, wobei als Initiatoren organische Peroxide wie Dibenzoylperoxid, Percarbonate wie z.B. Bis(4-tert.-Butylcyclohexyl)peroxidicarbonat oder besonders Azoverbindungen wie z.B. alpha,alpha'-Azoisobutyronitril (AIBN) geeignet sind.

Je nach Verwendungszweck, Substrat, Verarbeitungsgeschwindigkeit oder besonderen Anforderungen an das Ko- oder Adhäsionsvermögen des Schmelzhaftklebers kann es sinnvoll sein, während der Polymerisation das durchschnittliche Molekulargewicht des Polymerisats zu senken. Auch bei höheren Anteilen der unter b) genannten polaren Comonomere ist der Einsatz von Reglern vorteilhaft, da so die Schmelztemperatur gesenkt, bzw. die Fließfähigkeit im geschmolzenen Zustand verbessert wird. Für diesen Zweck eignen sich Verbindungen wie z.B. Dodecylmercaptan, Tetrabrommethan, Trichlorbrommethan oder auch Alkanole wie Ethanol oder Isopropanol.

Der Einsatz von N-tert.-Butylacrylamid als Comonomer zur Herstellung von strahlenvernetzbaren Acrylat-Schmelzhaftklebern beeinflußt die thermische und oxidative Stabilität der unter a) genannten acrylischen Monomere nicht nachteilig, so daß auf eine besondere Stabilisierung für den Schmelzvorgang verzichtet werden kann. Dennoch ist die Verwendung von geringen Mengen Antioxidantien möglicherweise von Vorteil , besonders, wenn die unter b) genannten Polaren Monomere in höheren Anteilen eingesetzt, oder auch wenn die Klebmassen längere Zeit bei höheren Temperaturen belassen werden. Als Stabilisatoren eignen sich u.a. sterisch gehinderte, arylische Thioether wie z.B. das IRGANOX 1035^{R} von CIBA-GEIGY (2,2'-Thiodiethylbis-(3,5-di-tert.butyl-4-hydroxyphenyl)-propionat) oder phenolische Verbindungen wie das SAN-TOWHITE PC^{R} (2,2-Methylen-bis-(4-Methyl-6-tert.Butylphenol)) von MONSANTO.

Der Anteil dieser Stabilisatoren sollte, je nach Typ und Polymerzusammensetzung, 0,50 Gew.-% bezogen auf die Menge an Polymer, vorzugsweise sogar 0,30 Gew.-% nicht übersteigen. Bei diesen Anteilen ist gewährleistet, daß die Vernetzbarkeit der erfindunsgemäßen Schmelzhaftkleber gegenüber energiereicher, ionisierender Strahlung, die durch Mischpolymerisation mit dem TBA erhalten wird, in vollem Umfang beibehalten wird.

Der vom Lösungsmittel befreite Acrylathaftkleber der vorliegenden Erfindung kann mit den üblichen Schmelzbeschichtungsanlagen wie Walzenauftragswerken, Ein- und Zweischneckenextrudern, Schmelz(rotations)siebdruck- oder auch einfachen Schlitzdüsenauftragswerken auf die Substrate aufgebracht werden, wobei das Mischpolymerisat beispielsweise Temperaturen von 60°C bis 200°C, vorzugsweise aber Temperaturen von 80°C bis 140°C, insbesondere 100 bis 120°C aufweist.

Die Klebmassen besitzen schon bei relativ niedrigen Temperaturen Schmelzviskositäten, die eine gute und sichere Verarbeitung gewährleisten, so daß sich auf diese Weise klare und fehlerfreie Klebefilme auf die unterschiedlichsten Substrate aufbringen lassen. Neben guter thermischer Beständigkeit zeigen sie auch hervorragende Fließeigenschaften, so daß es möglich ist, die Beschichtung von empfindlichen Substraten bei besonders niedrigen Temperaturen durchzuführen.

Gegenüber den bekannten Diacetonacrylamid-Copolymeren haben die erfindungsgemäßen TBA-Copolymere zudem noch den Vorteil einer größeren thermischen Stabilität. So führt das Copolymerisat aus 2-Ethylhexylacrylat und 10 Gew.-% Diacetonacrylamid schon nach vierstündiger Belastung bei üblicher Austragstemperatur (140°C) zum Gel, während bei dem mit TBA hergestellten Polymer noch keine Vergelungstendenzen festgestellt wurden.

Als Substrat können sowohl feste Unterlagen als auch blatt- oder bahnförmige, vorzugsweise flexible Materialien dienen. Auf diese Weise erhält man z.B. Klebefolien und Klebebänder für die technische oder medizinische Verwendung.

Die Auswahl an Beschichtungsmaterialien ist nicht beschränkt, geeignet sind sowohl gewebte, wie auch nichtgewebte Trägermaterialien. Zu den nichtgewebten Trägern zählen u.a. Folien aus Polyethylenterephthalat, Hart- und Weich-PVC, Polyethylen, Polypropylen oder auch Metallfolien. Als gewebte Substrate können Textilien oder Tuch aus beispielsweise Wolle, Baumwolle oder Seide zur Anwendung kommen.

Geeignet sind ebenso Trägermaterialien aus Papier, Pappe oder Cellulose. Darüber hinaus lassen sich auch geschäumte Materialien aus z.B. Polyurethanen oder Copolymerisate aus Ethylen/Vinylacetat mit den erfindungsgemäßen Schmelzhaftklebern auf Acrylatbasis beschichten.

Das Aufbringen der Klebmasse auf das Substrat kann sowohl indirekt mit dem sogenannten Transferverfahren, bei dem die Klebmasse erst auf einen Hilfsträger mit Trenneigenschaften aufgetragen und anschließend auf den eigentlichen Träger umkaschiert wird, wie auch direkt erfolgen.

Der hier verwendete Begriff "Beschichtung" beschränkt sich nicht auf die einseitige Beschichtung von Trägermaterialien, sondern schließt auch die beidseitige Ausrüstung von Substraten mit den Schmelzhaftklebemassen ein.

Die erfindungsgemäßen Schmelzhaftkleber können selbstverständlich auch Füllstoffe wie Aluminiumsilikat, Talcum, gefällte oder pyrogen gewonnene Kieselsäure, Glaskugeln, Glasfasern, Ruß, Titandioxid, Metallpulver u.a. enthalten; auch ist die Modifizierung mit verschiedenen Harzsystemen, z.B. Terpenphenolharze, möglich.

Die Dicke der Klebmasseschichten liegt vorzugsweise je nach Anwendungszweck zwischen 5 und 1500um, insbesondere zwischen 50 und 1000um, entsprechend einem Auftragsgewicht von 5 bis 1500 g/m², insbesondere zwischen 5 und 1000 g/m².

Für medizinische Verwendungen beträgt die Schichtdicke vorzugsweise 20 - 150µm, insbesondere 20 - 50µm, entsprechend einem Auftragsgewicht von 20 - 150 g/m² bzw. 20 - 50 g/m².

Zur Vernetzung der erfindungsgemäßen Schmelzhaftklebemassen eignen sich alle Arten energiereicher und ionisierender Strahlung wie Alpha-, Beta-, Gamma- und Röntgenstrahlung sowie UV-Strahlung nach Zufügen geeigneter Sensibilisatoren, beispielsweise IRGACURE^{R} von CIBA-GEIGY (z.B. IRGACURE^{R} 651, alpha,alpha-Dimethoxy-alpha-phenylacetophenon) oder DAROCUR^{R} von MERCK (z.B. DAROCUR^{R} 953, 1-(4-Dodecylphenyl)-2-hydroxy-methylpropan-1-on).

Photosensibilisatoren können in Mengen von vorzugsweise 0,5 - 7% in den Massen enthalten sein. Für den medizinischen Bereich werden mit UV-Strahlung vernetzte Schmelzhaftklebemassen bevorzugt. Als Strahlenquelle für Gammastrahlung dienen üblicherweise die Isotope Co⁶⁰ und Cs¹³⁷. Für die Erzeugung von β-Strahlen sind leistungsfähige Elektronenbeschleuniger entwickelt worden, die für diese Erfindung bevorzugt werden. Die Eindringtiefe dieser Strahlen und damit die maximale Stärke der zu vernetzenden Beschichtung wird von der Beschleunigerspannung bestimmt. Die absorbierte Strahlendosis wurde aus dem Strahlstrom und der Bahngeschwindigkeit berechnet. Die benutzten Funktionen sind an anderer Stelle veröffentlicht worden (W. Karmann, Coating 12/81, 365 (1981)).

Die Bestrahlung wird normalerweise unter Ausschluß von Luftsauerstoff vorgenommen, was aber für das Ergebnis nicht unbedingt erforderlich ist.

Zur Härtung der erfindungsgemäßen Acrylat-Schmelzhaftklebemassen sind Dosen der absorbierten Strahlung von 5 bis 250 kGy geeignet. Wegen der hohen Vernetzungsausbeute wird ein Bereich von 5 bis 100 kGy, insbesondere 10 bis 70 kGy bevorzugt. Besonders bevorzugt werden Dosen von 20 bis 50 kGy. Die UV-Vernetzung kann auf den üblichen Anlagen nach bekannten Verfahren erfolgen. Die Leistung der Quecksilberdampflampen beträgt vorzugsweise 60 - 100 W/cm. Bei einem Bestrahlungsabstand von ca. 15 cm liegen die Bahngeschwindigkeiten vorzugsweise zwischen 1 und 15 m/min.

Wie die nach Einwirkung von energiereicher, ionisierender Strahlung gestiegenen Zahlenwerte der Williamsplastizität zeigen, sind die durch Mischpolymerisation mit N-tert.-Butylacrylamid erhaltenen Schmelzhaftkleber sehr gut vernetzbar. Es werden durch Verwendung dieses Monomers Schmelzhaftkleber erhalten, die hohe Scherfestigkeiten aufweisen, und dies auch bei erhöhten Temperaturen.

Die Klebemassen weisen ein ausgewogenes Verhältnis zwischen adhäsivem und kohäsivem Verhalten auf, d.h., trotz hoher erreichbarer Kohäsion und Spaltfestigkeit fliegen sie gut auf die unterschiedlichsten Klebuntergründe auf und zeichnen sich danach durch hohe Ablöseadhäsion aus. In keinem Fall wurden Rückstände irgendwelcher Art auf den unterschiedlichsten Untergründen beobachtet, d.h., die nach Bestrahlung erhaltene Kohäsion der Klebemassen war stets größer als die Adhäsion auf dem Untergrund, obgleich diese in einigen Fällen beträchtlich hoch war.

Die Verankerung der gehärteten Klebesysteme ist sowohl auf unpolaren Trägern wie Polyethylenfolie oder Polypropylenfolie, wie auch auf ausgesprochenen polaren Substraten wie beispielsweise Cellulose oder PVC erstaunlich gut, so daß im allgemeinen kein Ablösen von gängigen Trägermaterialien erfolgt.

Mit Hilfe von energiereicher Strahlung, vorteilhaft ist die Anwendung von Elektronenstrahlen, läßt sich der Vernetzungsgrad der Klebemassen auf dem Substrat im gewünschten Maße einstellen, wobei durch das mischpolymerisierte N-tert.-Butylacrylamid nur relativ geringe Strahlendosen erforderlich sind, um zu druckempfindlichen Klebmassen mit hoher Kohäsionsfestigkeit zu gelangen, so daß diese Klebsysteme auch für technisch anspruchsvolle Anwendungen einsetzbar sind.

Diese Schmelzhaftklebemassen weisen eine hohe Alterungsbeständigkeit auch bei längerer Einwirkung von UV-Strahlung oder erhöhten Temperaturen auf: Nach Vernetzung durch Bestrahlung werden Änderungen im Klebverhalten der mit den erfindungsgemäßen Acrylat-Schmelzhaftklebern beschichteten Trägermaterialien praktisch nicht mehr beobachtet.

Durch die Verwendung von N-tert.-Butylacrylamid (TBA) als Comonomer behalten die erfindungsgemäß in Acrylhaftklebern eingesetzten Hauptmonomere wie zum Beispiel n-Butyl-, Hexyl-, Octyl- und iso-Octyl-, 2-Ethylhexyl-, Decyl- oder iso-Decylacrylat bzw. -methacrylat ihr druckempfindliches Selbstklebverhalten bei. Zusätzlich zeigt sich aber eine gesteigerte Vernetzungsausbeute durch energiereiche Strahlung, so daß sich durch Mischpolymerisation der (meth)acrylischen Monomeren mit TBA strahlenvernetzbare Schmelzhaftklebstoffe herstellen lassen, die über eine ausgezeichnete Kohäsion, bei gleichzeitig gutem Tack und hohem Adhäsionsvermögen verfügen.

Diese Eigenschaften werden sowohl in trockener, wie auch in feuchter Umgebung erhalten. Auch in letzterem Fall weisen mit N-tert.-Butylacrylamid hergestellte Schmelzhaftklebemassen ein gutes und dauerhaftes Hautverklebungsverhalten auf, die sie unter anderem für die Anwendung im medizinischen Bereich geeignet machen, beispielsweise für Pflaster, z.B. Heftpflaster, Wundpflaster, haftende Binden, Klebebänder, Klebefolien, z.B. Inzisionsfolien und Stoma-Versorgungen. Für medizinische Zwecke läßt sich die erfindungsgemäße Schmelzhaftklebemasse vorteilhafterweise mit antimikrobiellen Wirkstoffen versehen. Der Anteil kann etwa 0,1 bis 30 Gew.-% bezogen auf das Gesamtgewicht betragen. Solche Wirkstoffe für Selbstklebemassen sind in der Literatur beschrieben. Auch diese Verwendungen sind Gegenstand der Erfindung.

Die so hergestellten Klebematerialien benötigen zwar nicht die hohen Klebkräfte und Scherfestigkeiten technischer Klebebänder, bieten aber alle Vorteile, die von einem medizinischen Klebeband erwartet werden, das für eine Verklebung auf der Haut eingesetzt wird. Hierzu zählen besonders die gute Verträglichkeit auf der Haut - auch bei sensibeleren Personen traten keine Irritationen auf-und kein zu agressives Klebeverhalten: Die Probestreifen ließen sich trotz dauerhaftem Sitz auf der Haut auch wieder gut hiervon ablösen, ohne daß es zu einem starken Abstoßen von oberen Hautschichtpartikeln kam. Die mit diesen Klebmassen ausgerüsteten Pflaster wiesen nur eine sehr geringe Neigung zum Umspulen auf die Haut auf und dies trotz der nur geringen angewendeten Strahlendosis.

Die erfindungsgemäßen Acrylat-Schmelzhaftkleber weisen mindestens alle gewünschten Eigenschaften einer üblicherweise aus Lösungsmitteln aufgetragenen Klebemasse auf, d.h. zum Beispiel die Ausgewogenheit zwischen kohäsivem und adhäsivem Verhalten. Sie bietet außerdem den Vorteil, daß bei der Verarbeitung auf technisch aufwendige und kostenintensive Trocknungsanlagen verzichtet werden kann.

Die verwendeten Stoffe sind bekannt oder im Handel erhältlich.

Alle Mengenangaben, Anteile und Prozentanteile sind, soweit nicht anders angegeben, auf das Gewicht und die Gesamtmenge an Monomeren bezogen.

Gegenstand der Erfindung ist auch die Verwendung von blatt- oder bahnförmige Materialien mit einem Auftrag eines Schmelzhaftklebers, der nach dem Verfahren gemäß Anspruch 1 erhältlich ist im medizinischen Bereich.

Die oben genannten beschichteten blatt- und bahnförmigen Träger können in Streifen geschnitten und zu Klebebandrollen aufgewickelt werden.

Zur Beurteilung der erfindungsgemäßen Acrylat-Schmelzhaftkleber wurden nachfolgende Prüfmethoden herangezogen:

### K-Wert:(nach FIKENTSCHER)

Der K-Wert ist ein Maß für die durchschnittliche Molekülgröße hochpolymerer Stoffe. Zur Messung wurden einprozentige (1 g/100 ml) toluolische Polymerlösungen hergestellt und mit Hilfe eines VOGEL-OSSAG-Viskosimeters deren kinematischen Viskositäten bestimmt. Nach Normierung auf die Viskosität des Toluols wird die relative Viskosität erhalten, aus der sich nach FIKENTSCHER der K-Wert errechnen läßt (Polymer 8/1967, 381 ff).

### Schälfestigkeit (Klebkraft, KK):

Zur Messung der Klebkräfte wurden 19 mm breite Prüfstreifen blasenfrei auf eine fein geschliffene (Schmirgelpapier mit FEPA-Körnung 240) Stahlplatte aus rostfreiem Stahl geklebt und mit einer gummiumwickelten 2-kg-Rolle mit einer Geschwindigkeit von 10 m/min angedrückt. Die Stahlplatte und das überstehende Ende des Klebebandes wurden dann in der Weise in die Enden einer Zugprüfmaschine gespannt, daß ein Abzugswinkel von 180° entstand. Das Klebeband wurde mit einer Geschwindigkeit von 300 mm/min von der Stahlplatte abgezogen. Die Angabe der Klebkraft erfolgt in N/cm.

### Scherstandzeit (Holding-Power, HP):

Die Scherstandzeit gibt die Verklebungsfestigkeit für eine parallel zum verklebten Band angreifende Belastungskraft an. Sie ist die Zeit, die bis zum völligen Abscheren eines belasteten Klebebandes von einer Stahlplatte gemessen wird.

Zur Bestimmung der HP-Werte wird ein 19 mm breiter Prüfstreifen so auf ein vorbehandeltes (s. 180 °-Ablöseadhäsion) Stahlplättchen geklebt, daß eine Verklebungsfläche von 19x20 mm² entsteht. An das überstehende Ende des Klebebandes wird mit Hilfe einer Klammer ein 1-kg-Gewicht gehängt, so daß eine senkrechte Zugkraft von 5,15 N pro 1 cm Bandbreite übertragen wird.
Die Messungen wurden bei Raumtemperatur (Rt = 22°C) und z.T. auch bei 80°C durchgeführt. Die Einheit der Scherstandzeit ist Minuten. Steht vor den Werten ein ">"-Zeichen, bedeutet das, daß die Messungen nach dieser Zeit abgebrochen wurden, da noch keinerlei Scheren zu erkennen war. "(K)" bedeutet Kohäsionsbruch mit Masserückständen, "(A)" ist die Abkürzung für Adhäsionsbruch.

### Schmelzviskosität Eta*:

Zur Charakterisierung der erfindungsgemäßen Acrylat-Schmelzhaftkleber wurde neben den oben genannten Klebeigenschaften auch das Fließverhalten der unvernetzten Klebemassen im geschmolzenen Zustand herangezogen. Die Bestimmung erfolgte nach einer oszillierenden Methode mit Hilfe eines "RHEOMETRIC DYNAMIC SPECTROMETERs" (RDS).

Als konstante Randbedingungen wurden eine Prüftemperatur von 140°C, eine Normalkraft von 5% (=50 g) und eine Amplitude von 5% der maximalen Auslenkbarkeit (=0,5 rad) gewählt. Die Angabe der hier ermittelten komplexen, dynamischen Schmelzviskosität Eta* gilt für eine Umfanggeschwindigkeit von w = 10 rad/s, entsprechend einem Schergefälle von etwa γ̇ = 10/s (Fließverhalten von Stoffen und Stoffgemischen, W.-M. Kulicke, Verlag Hüthig & Weps, 1986, S. 88 ff).

### Williamsplastizität (W.P.):

Bei hoher Vernetzungsausbeute mit Ausbildung eines dreidimensionalen Netzwerkes weisen Polymere nur noch geringe Plastizität auf und ihre Neigung zum "kalten Fließen" geht zurück. Durch Vergleich der Williamsplastizitäten vor und nach der Bestrahlung mit energiereichen Strahlen läßt sich die Kohäsionssteigerung der Klebemassen quantifizieren.

Zur Messung werden 2 g eines Polymers zu einer Kugel geformt und nach ausreichender Temperierung zwischen die parallelen Platten eines Williams-Plastometers gelegt. Danach wird die Polymerprobe bei 38°C 14 min mit 5000 g belastet. Die Angabe der Williamsplastizität erfolgt in mm , d.h., große Werte bedeuten eine hohe Vernetzung.

### Gelwert:

Eine weitere Größe zur Bestimmung des Vernetzungsumfangs ist der sogenannte Gelwert; dies ist der Anteil an Polymer, der sich nach 24-stündiger Extraktion in Toluol bei Raumtemperatur nicht gelöst hat.

Die nachfolgenden Beispiele sollen einen Eindruck vermitteln, wie leistungsfähig die neuen Acrylat-Schmelzhaftkleber sind. Aus der Vielzahl der geeigneten Beschichtungsuntergründe wurden stellvertretend folgende Materialien ausgewählt (Tabelle I):

| Code | Substrat |
|---|---|
| I | Naß-Vlies aus Cellulose-Viskose 20/80, Dicke: 225 µm, Flächengewicht: 30 g/m². |
| II | Cellulose-Gewebe, Fadenzahl: längs≥66, quer≥25, Flächengewicht: 95 g/m². |
| III | Folie aus Polyethylen (PE), druckvorbehandelt, Flächengewicht: 110 g/m², Dicke: 120 µm. |
| IV | 25 µm Folie aus Poyethylenterephtalat (PETP), Flächengewicht: 35 g/m², zur Haftverbesserung mit einem Vorstrich aus Polyvinylidenchlorid ausgerüstet. |
| V | Biaxialgereckte Polypropylen-Folie, durckvorbehandelt, Flächengewicht: 30 g/m², Dicke: 30 µm. |
| VI | Coronavorbehandelter Schaum aus Ethylen/Vinylacetat-Copolymer (EVA), Rohdichte: 200 kg/m³. |
| VII | Polyethylen-Vlies, Flächengewicht: 55 g/m², Dicke: 140 µm. |

In den nachfolgend beschriebenen Beispielen erfolgt die Substratbeschichtung mit einem Schlitzdüsenauftragswerk. Die Haftkleber werden in einer 2-kg-Patrone mit Heizmantel aufgeschmolzen und unter einem Druck von 4 bar durch eine ebenfalls beheizbare Düse auf das Substrat gedrückt.

Die erfindungsgemäßen Schmelzhaftkleber der nachfolgenden Beispiele werden mit einem Elektronenstrahler des Scanner-Typs der Firma HIGH-VOLTAGE ENGINEERING CORP., Burlington, betrahlt. Die Beschleunigerspannung beträgt 350 kV und der Strahlstrom variiert zwischen 10 und 50 mA. Alle Bestrahlungen werden unter einer N₂-Inertgas Atmosphäre durchgeführt.

### BEISPIEL 1-3

In einen 2-I-Glasreaktor mit Ankerrührwerk, Rückflußkühler, Thermometer und Gaseinleitungsrohr werden 400 g Monomerenmischung, bestehend aus 95 Gewichtsteilen 2-Ethylhexylacrylat (EHA) und 5 Gewichtsteilen N-tert. Butylacrylamid, und je 133 g Aceton und Benzin (Siedebereich 60-95°C) eingefüllt. Nach 30-minütigen Spülen der Apparatur mit Stickstoff wird mit einer Badtemperatur von 75°C aufgeheizt. Um die Polymerisation einzuleiten, werden bei einer Innentemperatur von 60°C 0,20 g AIBN zu der Reaktionsmischung zugegeben. Eine Stunde nach Reaktionsstart, der durch eine starke Exothermie und refluxierende Lösungsmittel zu erkennen ist, werden dem Ansatz weitere 0,20 g AIBN zugegeben.

Die Reaktionszeit beträgt insgesamt 24 Stunden, wobei die Badtemperatur nach 4 Stunden von 75 auf 65°C abgesenkt wird. Erforderliche Verdünnungen werden so mit dem oben genannten Benzin vorgenommen, daß ein Endfeststoffgehalt von 45% erhalten wird. Nach beendeter Reaktion werden in der Polymermischung 1,2 g des Antioxidants IRGANOX 1035 (CIBA-GEIGY) gelöst, danach wird das Lösungsmittel im Vakuum abgezogen.

Der auf diese Weise hergestellte Acrylat-Schmelzhaftkleber hat einen K-Wert von 74 und eine Schmelzviskosität von 280 Pas. Die Williamsplastizitäten (W.P.) und Gelanteile für die verschiedenen Strahlendosen zeigt Tabelle II.

**Tabelle II:**

| W.P. und Gelanteile für ein Polymer aus EHA/TBA i.V. 95/5. | | | |
|---|---|---|---|
| | Dosis [kGy] | W.P. [mm] | Gelanteil [%] |
| | 0 | 1,02 | 0 |
| | 30 | 1,74 | 45 |
| | 50 | 1,98 | 66 |
| | 70 | 2,43 | 72 |

In Tabelle III sind die für die verschiedenen Substrate und Strahlendosen erhaltenen Klebkräfte aufgeführt.

**Tabelle III:**

| Beispiel | Substrat | Masseauftrag [g/m²] | Dosis [kGy] | Klebkraft [N/cm] |
|---|---|---|---|---|
| 1 | I | 40 | 30 | 2,50 |
| 2 | II | 90 | 50 | 2,15 |
| 3 | II | 90 | 70 | 1,90 |

### BEISPIEL 4-10

Die Polymerisationen erfolgten analog den Beispielen 1 bis 3, mit der Maßgabe, daß in diesen Beispielen 2-Ethylhexylacrylat und TBA in einem Verhältnis von 90 zu 10 Gewichtsteilen eingesetzt wurden. Die Williamsplastizitäten und Gelanteile vor und nach Elektronenbestrahlung zeigt Tabelle IV.

**Tabelle IV:**

| W.P. und Gelanteile für ein Monomerenverhältnis von EHA/TBA zu 90/10. | | |
|---|---|---|
| Dosis [kGy] | W.P. [mm] | Gelwert [Gew.%] |
| 0 | 1,36 | 0 |
| 30 | 2,40 | 51 |
| 50 | 2,70 | 68 |
| 70 | 2,91 | 76 |

Der K-Wert dieses Schmelzhaftklebertyps beträgt 82, die Schmelzviskosität 490 Pas. Beschichtet wurde in allen Beispielen bei einer Massetemperatur von 140°C. In Tabelle V sind die für die unterschiedlichen Substrate und Vernetzungsdosen der Beispiele 4-10 erhaltenen Klebkraftwerte aufgeführt.

**Tabelle V:**

| Klebkräfte für den Schmelzhaftkleber EHA/TBA=90/10 der Beispiele 4 bis 10. | | | | |
|---|---|---|---|---|
| Beispiel | Substrat | Masseauftrag [g/m²] | Dosis [kGy] | Klebkraft [N/cm] |
| 4 | I | 55 | 30 | 2,50 |
| 5 | III | 55 | 40 | 2,20 |
| 6 | III | 50 | 30 | 1,70 |
| 7 | III | 60 | 40 | 1,90 |
| 8 | VII | 20 | 20 | 3,00 |
| 9 | VII | 20 | 30 | 2,80 |
| 10 | VII | 20 | 50 | 2,75 |

Die in den Beispielen 4 bis 10 eingesetzte Schmelzhaftklebemasse zeigte ein ausgesprochen gutes Hautverklebungsverhalten, das besonders diese Monomerkombination für strahlenhärtbare Pflasterklebemassen bzw. für Klebebänder zur Verbandsfixierung geeignet macht: Auch bei Einwirkung von Feuchtigkeit hielten die Substrate gut auf der Haut, erzeugten keine Irritationen und ließen sich selbst nach längerer Verklebungszeit wieder gut von der Haut entfernen, ohne daß oberste Hautschichten mit abgelöst wurden.

### BEISPIEL 11-16

Entsprechend den Beispielen 1 bis 3 wurde eine Klebmasse hergestellt, deren Monomere 2-Ethylhexylacrylat und N-tert. Butylacrylamid im Verhältnis von 80 zu 20 Gewichtsteilen eingesetzt wurden. Die gute Strahlenvernetzbarkeit wird durch die Steigerung der Williamsplastizität zum Ausdruck gebracht: Während das unbestrahlte Copolymer eine W.P. von 1,98 aufwies, stieg sie nach Härtung mit 30 kGy auf 2,42, nach Bestrahlung mit 50 kGy auf 2,87, und die Einwirkung einer Elektronenstrahl-Dosis von 70 kGy ließ die Williamsplastizität sogar auf 3,45 steigen.
Die Schmelzviskosität dieser Klebmasse betrug 510 Pas, und der K-Wert wurde zu K=82 bestimmt. Beschichtet wurden eine Polyester- und Polypropylenfolie mit 25 g/m², sowie ein EVA-Schaum mit 65 g/m² Masseauftrag. Die Beschichtungstemperatur betrug 140°C.

In Tabelle VII sind die für die unterschiedlichen Substrate und Strahlendosen gemessenen klebtechnischen Daten aufgelistet.

**Tabelle VII:**

| | Beispiel | Substrat | Dosis [kGy] | KK [N/cm] | Scherstandzeit in min | |
|---|---|---|---|---|---|---|
| | | | | | Rt | 80°C |
| | 11 | IV | 30 | 4,05 | > 15000 | 30 (K) |
| | 12 | IV | 50 | 3,85 | > 15000 | > 15000 |
| | 13 | IV | 70 | 3,65 | > 15000 | 600 (A) |
| | | | | | | |
| | 14 | V | 40 | 3,00 | > 15000 | - |
| | 15 | V | 50 | 2,90 | > 15000 | - |
| | | | | | | |
| | 16 | VI | 70 | 4,90 | - | - |
| | | | | | | |

Durch Mischpolymerisation von 2-Ethyhexylacrylat mit 20 Gewichtsteilen N-tert. Butylacrylamid werden Schmelzhaftkleber mit ausgeprägten kohäsiven Eigenschaften erhalten. Schon mit der geringen Strahlendosis von 30 kGy (Beispiel 11) lassen sich äußerst scherfeste Schmelzhaftklebemassen herstellen, bei denen nach 15000 min bei Raumtemperatur noch keinerlei Scherung zu erkennen ist. Diese hohe innere Festigkeit geht dabei aber nicht zu Lasten des Adhäsionsvermögens, es werden auf PETP-Folie je nach Bestrahlungsdosis Klebkräfte zwischen 3,65 und 4,05 N/cm, und bei Verwendung der PP-Folie Adhäsionswerte von ca. 3 N/cm erhalten.

### BEISPIEL 17-19

Für die Beispiele 17 bis 19 wurde eine Monomerenmischung aus 69 Gewichtsteilen 2-Ethylhexylacrylat, 20 Gewichtsteilen n-Butylacrylat (BA), 10 Gewichtsteilen N-tert. Butylacrylamid und 1 Gewichtsteil Acrylamid (AA) analog den Beispielen 1 bis 3 hergestellt. Der mit diesem Monomerenverhältnis hergestellte Acrylatschmelzhaftkleber hat einen K-Wert von K=83,5, und die Schmelzviskosität beträgt 600 Pas. Die Vernetzbarkeit dieses Systems wird wiederum durch die hohen Werte der Williamsplatizitäten und Gelanteile nach Elektronenbestrahlung in Tabelle VIII dokumentiert.

**Tabelle VIII:**

| W.P. und Gelanteile vor und nach Bestrahlung für einen Acrylatschmelzhaftkleber, bestehend aus EHA/BA/TBA/AA i.V. 69/20/10/1. | | |
|---|---|---|
| Dosis [kGy] | W.P. [mm] | Gelanteil [Gew.%] |
| 0 | 1,82 | 0 |
| 30 | 2,42 | 54 |
| 50 | 2,71 | 71 |
| 70 | 3,04 | 81 |

Die Schmelzmasse wurde bei 140°C mit einem Masseauftrag von 20 g/m² auf PETP-Folie (IV) ausgetragen. Die nach Vernetzung erhaltenen Klebkraft- und Holding-Power-Werte sind in Tabelle IX zusammengestellt.

**Tabelle IX:**

| Klebkräfte und Scherfestigkeiten des Schmelzhaftklebers der Beispiele 17-19. | | | | |
|---|---|---|---|---|
| Beispiel | Dosis [kGy] | Klebkraft [N/cm] | Scherstandzeit in min | |
| | | | Rt | 80°C |
| 17 | 30 | 2,90 | 2650(A) | 90(K) |
| 18 | 50 | 2,80 | > 20000 | > 20000 |
| 19 | 70 | 2,55 | 19000(A) | > 20000 |

Es zeigt sich, daß für diesen Schmelzhaftkleber die optimale Vernetzungsdosis zwischen 30 und 70 kGy liegt: Nach Härtung mit 50 kGy wurde bei den Scherfestigkeitsmessungen bei Rt noch nach 20000 min keine Scherung festgestellt, während die mit 70 kGy bestrahlten Muster im Durchschnitt bereits nach 19 000 min Adhäsionsbruch zeigten. Bei einer Temperatur von 80°C wiesen sowohl die 50 kGy-, wie auch die 70 kGy-Muster außerordentlich hohe Scherfestigkeiten auf; in beiden Fällen war selbst nach 20000 min noch keinerlei Scherung erkennbar.

### BEISPIEL 20-22

Für die Beispiele 20 bis 22 wurde ein Terpolymerisat verwendet, das ausschließlich aus Acrylsäureestern und TBA hergestellt wurde. Es handelt sich hierbei also um einen Schmelzhaftkleber, der zusätzlich zum TBA kein weiteres modifizierendes, polares Comonomer enthält. Die Reaktionsmischung besteht aus 80 Gewichtsteilen 2-Ethylhexylacrylat, 10 Gewichtsteilen N-tert. Butylacrylamid und 10 Gewichtsteilen Isobornylacrylat (IBOA), wobei die Herstellung analog den Beispielen 1 bis 3 erfolgte. Der K-Wert der Klebemasse beträgt K = 76, und für die Schmelzviskosität wurde ein Wert von 340 Pas ermittelt.

Trotz recht geringer Williamsplastizität im unvernetzten Zustand wurden nach Elektronenbestrahlung wieder hohe W.P.-Werte und Gelanteile gemessen (Tabelle X).

**Tabelle X:**

| Williamsplastizitäten und Gelanteile für den verwendeten Acrylat-Schmelzhaftkleber der Beispiele 20-22. | | |
|---|---|---|
| Dosis [kGy] | W.P. [mm] | Gelanteil [Gew.%] |
| 0 | 1,29 | 0 |
| 30 | 2,17 | 48 |
| 50 | 2,55 | 66 |
| 70 | 2,73 | 75 |

Der vom Lösungsmittel befreite Haftkleber der Beispiele 20 bis 22 wurde bei einer Temperatur von 145°C aus der Schlitzdüse mit 30 g/m² Masseauftrag auf eine PETP-Folie (Substrat IV) aufgebracht. Nach Vernetzung-mit Strahlendosen zwischen 30 und 70 kGy wurden Klebkräfte und Scherfestigkeiten gemessen, deren Ergebnisse in Tabelle XI zusammengestellt sind.

**Tabelle XI:**

| Klebkräfte und Scherfestigkeiten für die Schmelzhaftkleber der Beispiele 20 - 22. | | | |
|---|---|---|---|
| Beispiel | Dosis [kGy] | Klebkraft [N/cm] | HP/Rt [min] |
| 20 | 30 | 4,3 | 1670 (K) |
| 21 | 50 | 3,9 | 2260 (A) |
| 22 | 70 | 3,3 | 2325 (A) |

### BEISPIEL 23-26

Für die Beispiele 23 bis 26 wurde ein Polymerisat verwendet, das 2-Ethylhexylacrylat in 87, TBA in 10, und Acrylsäure (AS) in 3 Gewichtsanteilen enthielt. Zur Herstellung wurde wie in den Beispielen 1 bis 3 vorgegangen. Zur Verringerung des durchschnittlichen Molekulargewichts wurden dem Ansatz vor Polymerisationsbeginn zusätzlich noch 8 g Isopropanol zugesetzt, so daß eine Klebemasse mit einem K-Wert von K = 65 entstand. Die Schmelzviskosität wurde zu 380 Pas bestimmt.

Das plastische Verhalten und die Vernetzbarkeit dieses Systems wurde wiederum über die Williamsplastizitäten und Gelanteile bestimmt, dessen Werte in Tabelle XII aufgeführt sind.

**Tabelle XII:**

| Gelwerte und Williamsplastzitäten des Schmelzhaftklebers der Beispiele 23-26. | | |
|---|---|---|
| Dosis [kGy] | W.P. [mm] | Gelanteil [Gew.%] |
| 0 | 1,71 | 0 |
| 30 | 2,46 | 55 |
| 50 | 2,79 | 70 |
| 70 | 3,19 | 77 |

Beschichtet wurden Polyester- und PP-Folie mit 25 g/m²-Masseauftrag, wobei sich zeigte, daß diese Monomerenkombination zu einem Schmelzhaftkleber führt, der über eine hohe Kohäsivität verfügt, und dies sogar bei erhöhten Temperaturen. Die nach Vernetzung mit energiereichen Elektronenstrahlen erhaltenen Klebkraft- und Scherstandzeitwerte sind in Tabelle XIII zusammengefaßt.

**Tabelle XIII:**

| Klebeigenschaften des Schmelzhaftkleber der Beispiele 23 bis 26. | | | | | |
|---|---|---|---|---|---|
| Bspl. | Substrat | Dosis [kGy] | Klebkraft [N/cm] | Scherstandzeit in min | |
| | | | | Rt | 80°C |
| 23 | IV | 30 | 3,55 | > 20000 | 220 (K) |
| 24 | IV | 50 | 3,45 | > 20000 | > 15000 |
| 25 | IV | 70 | 3,30 | > 20000 | > 15000 |
| 26 | V | 40 | 3,40 | > 20000 | - |

In diesen Beispielen wird wiederum deutlich, wie strahlensensibel diese neuartigen Schmelzhaftkleber sind: schon nach Bestrahlung mit 30 kGy wurden bei Raumtemperatur Scherstandzeiten von über 20000 min gefunden. Und nach Härtung ab 50 kGy wurden selbst nach 15000 Minuten bei 80°C noch keinerlei Scherung festgestellt.

### BEISPIEL 27-33

Die Monomerenmischung des Schmelzhaftklebers der Beispiele 27 bis 33 besteht aus 87 Gewichtsteilen 2-Ethylhexylacrylat, 10 Gewichtsteilen TBA und 3 Gewichtsteilen Maleinsäureanhydrid. Die Polymerisation wurde analog den Beispielen 1 bis 3 durchgeführt. Tabelle XIV zeigt die Gelwerte und Williamsplastizitäten vor und nach Bestrahlung.

**Tabelle XIV :**

| Dosis [kGy] | Gelanteil [Gew.%] | W.P. [mm] |
|---|---|---|
| 0 | 0 | 1,03 |
| 30 | 33 | 1,35 |
| 50 | 63 | 2,27 |
| 70 | 77 | 2,51 |

Der K-Wert des hier beschriebenen Schmelzhaftklebers beträgt K = 53,7. Die Schmelzviskosität ist außerordentlich gering und beträgt nur 40 Pas. Zur Beschichtung der Substrate wurde in diesen Beispielen eine Klebemassetemperatur von 90°C gewählt. Trotz der milden Austragsbedingungen wurde ein fehlerfreier Massefilm erhalten. Die Ergebnisse der klebtechnischen Prüfungen sind in Tabelle XV zusammengestellt.

**Tabelle XV :**

| Bspl. | Substrat | Masseauftrag [g/m²] | Dosis [kGy] | Klebkraft [N/cm] | HP / Rt [min] |
|---|---|---|---|---|---|
| 27 | IV | 30 | 30 | 4,60 | 865(K) |
| 28 | IV | 30 | 50 | 4,10 | 15000(K) |
| 29 | IV | 30 | 70 | 4,00 | > 20000 |
| 30 | IV | 60 | 50 | 5,20 | 9700(A) |
| 31 | IV | 60 | 70 | 4,40 | > 15000 |
| 32 | V | 30 | 40 | 3,80 | 10000(A) |
| 33 | V | 30 | 50 | 3,80 | > 15000 |

### VERGLEICH A und B

Um aufzuzeigen, welchen erheblichen Einfluß das N-tert. Butylacrylamid auf die Vernetzungsfähigkeit und die daraus resultierenden Klebeigenschaften hat, wurden in den Beispielen A und B TBA-freie Schmelzhaftkleber hergestellt. Während in Vergleich A ein 2-Ethylhexylacrylat(EHA)-Homopolymer eingesetzt wurde, bestand der Vergleich B aus einem Copolymer aus EHA und Acrylsäure im Gewichtsverhältnis 99 zu 1. Polymerisiert wurde analog den Beispielen 1 bis 3, der K-Wert betrug in beiden Fällen K = 70. Tabelle A zeigt die vor und nach Bestrahlung erhaltenen Williamsplastizitäten und Gelanteile.

**Tabelle A:**

| Williamsplastizitäten und Gelanteile der Vergleichsbeispiele ohne TBA. | | | | |
|---|---|---|---|---|
| Dosis [kGy] | W.P. in mm | | % Gel | |
| | Vgl. A | Vgl. B | Vgl. A | Vgl. B |
| 0 | 0,81 | 0,92 | 0 | 0 |
| 30 | 1,45 | 1,96 | 20 | 52 |
| 50 | 1,50 | 2,39 | 46 | 70 |
| 70 | 2,34 | 2,43 | 56 | 75 |

Die Schmelzviskositäten wurden zu 260 Pas (A) bzw. 330 Pas für Vergleich B bestimmt. Die erreichten klebtechnischen Werte dieser Vergleichsklebemassen sind in Tabelle B zusammengefaßt. Beschichtet wurde Polyesterfolie (Substrat IV) mit einem Masseauftrag von 20 g/m² (Vgl. A), bzw. 28 g/m² beim Vergleichsbeispiel B.

**Tabelle B:**

| Klebeigenschaften der Vergleichsbeispiele A und B. | | | | |
|---|---|---|---|---|
| | Dosis [kGy] | Klebkraft [N/cm] | Scherstandzeit in min | |
| | | | Rt | 80°C |
| | 30 | *) | 5 (K) | - |
| Vgl. A | 50 | 2,5 | 130 (K) | - |
| | 70 | 1,7 | 250 (K) | - |
| | 30 | 2,4 | 245 (K) | 20 (K) |
| Vgl. B | 50 | 1,8 | 300 (K) | 90 (K) |
| | 70 | 1,4 | 200 (A) | 25 (A) |

| | | | | |
|---|---|---|---|---|
| *) Die mit 30 kGy bestrahlte Vergleichsmasse A war so unvollständig vernetzt, daß bei den Messungen der Klebkräfte Masserückstände auf der Stahlschiene auftraten. | | | | |

### Beispiel 34 - 36

Für die UV-Vernetzung der Beispiele 34 - 36 wurde das Copolymer aus 90 Gewichtsteilen 2-Ethylhexylacrylat und 10 Gewichtsteilen N-tert.-Butylacrylamid ausgewählt. Die Herstellung des Schmelzhaftklebers erfolgte analog den Beispielen 1 bis 3, zusätzlich wurde die Masse aber noch mit 1,0% IRGACURE 651 (Ciba-Geigy, CH) bezogen auf den Feststoff als Photosensibilisator abgemischt. Die Schmelzbeschichtung erfolgte analog den Beispielen 4 - 10 auf einem Naß-Vlies (Substrat I) mit 40 g/m² Masseauftrag .

Die Vernetzung erfolgte mittels einer Quecksilberdampflampe, die eine Leistung von 80 W/cm aufwies. Die beschichtete Bahn wurde in einem Abstand von 150 mm mit Geschwindigkeiten (V) zwischen 2 und 10 m/min. unter der Strahlungsquelle entlanggeführt.

Die Tabelle XVI zeigt die erhaltenen Ergebnisse:

| Beispiel | (V) (m/min) | W.P. (mm) | Gelwert (Gew.-%) | Klebkraft (N/cm) |
|---|---|---|---|---|
| 34 | 2 | 2,61 | 65 | 2,25 |
| 35 | 5 | 2,15 | 40 | 2,55 |
| 36 | 10 | 1,78 | 20 | 3,80 |

## Patentansprüche

1. Verfahren zur Herstellung von Schmelzhaftklebern auf Acrylatbasis durch Bestrahlung auf einem Substrat, dadurch gekennzeichnet, daß man
a) 55 bis 98 Gew.-% mindestens eines acrylischen Monomers mit 2 bis 30 Gew.-% N-tert.-Butylacrylamid (TBA) copolymerisiert, wobei
b) die zu polymerisierende Reaktionsmischung (von a) zusätzlich eine oder mehrere ethylenisch ungesätigte Verbindungen enthalten kann, die nicht der Gruppe der acrylischen Monomere von a) angehören und wobei die zu polymerisierende Reaktionsmischung (von a) keine UV-vernetzbare Massen auf Basis von (Meth)acrylester-Copolymerisaten eines K-Werts von 15 bis 100, mit einem Gehalt von 0.01 bis 10 Gew.%, bezogen auf die Copolymerisate an einpolymerisierten Monomeren der allgemeinen Formel I 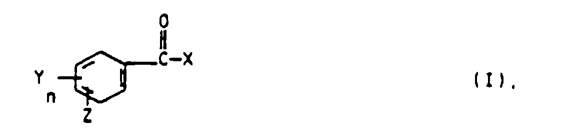 in der
X für eine 1 bis 3 C-Atome enthaltende Alkylgruppe oder einen gegebenenfalls durch n-Gruppen Y substituierten Phenylrest,
Y für -H, -CF₃, -O-Alkyl- und/oder Alkyl-COO- mit jeweils 1 bis 4 C-Atomen in der Alkylgruppe, Halogen, -CN, -COOH oder eine nicht ortho-ständige -OH-Gruppe,
n für 0 bis 4 und
Z für eine Gruppe der allgemeinen Formel 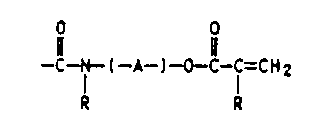 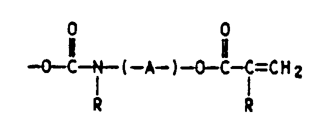 oder 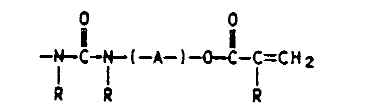 steht, in der
R H oder C₁- bis C₄-Alkyl und
A einen 2 bis 12 C-Atome aufweisenden Alkylen-, Oxaalkylen-oder Polyoxaalkylenrest bedeuten,
enthält,
c) das erhaltene feste Polymerisat erwärmt und in fließfähiger oder flüssiger Form auf ein Substrat aufbringt, wobei die Massen der Schritte a) bis c) gegebenenfalls Zusatz- und Hilfsstoffe enthalten können, und
d) das beschichtete Substrat mit energiereicher und ionisierender Strahlung bestrahlt.

2. Verfahren nach Anspruch 1, dadurch gekennzeichnet, daß das acrylische Monomer a) ein Alkylester der Acrylsäure oder Methacrylsäure ist, wobei der Alkylrest 1 bis 18 C-Atome besitzt.

3. Verfahren nach Anspruch 1, dadurch gekennzeichnet, daß die ethylenisch ungesättigten Verbindungen polare Gruppen enthalten.

4. Verfahren nach Anspruch 1, dadurch gekennzeichnet, daß der Anteil der ethylenisch ungesättigten Verbindungen 1 bis 15 Gew.-% beträgt, bezogen auf die Gesamtmenge der in der Mischung enthaltenen Monomeren.

5. Verfahren nach Anspruch 1, dadurch gekennzeichnet, daß das Mischpolymerisat bei Temperaturen von 60 bis 200°C auf ein Substrat aufgebracht wird.

6. Verfahren nach Anspruch 1, dadurch gekennzeichnet, daß die Dosis der energiereichen Strahlung 5 bis 250 kGy beträgt.

7. Verwendung von blatt- oder bahnförmigen Materialien mit einem Auftrag eines Schmelzhaftklebers, der nach dem Verfahren gemäß Anspruch 1 erhältlich ist, zur Herstellung von Klebematerialien im medizinischen Bereich.

## Claims

1. Process for the preparation of acrylate-based hot-melt pressure-sensitive adhesives by irradiation on a substrate, characterised in that
a) 55 to 98% by weight of at least one acrylic monomer is copolymerised with 2 to 30% by weight of N-tert.-butylacrylamide (TBA), wherein
b) the reaction mixture (of a) to be polymerised can additionally contain one or more ethylenically unsaturated compounds which do not belong to the group of acrylic monomers of a), the reaction mixture (of a) to be polymerised comprising no
UV-crosslinkable compositions based on (meth)acrylic ester copolymers with a K value of 15 to 100 containing from 0.01 to 10% by weight, based on the copolymers, of copolymerized monomers of the general formula (I) 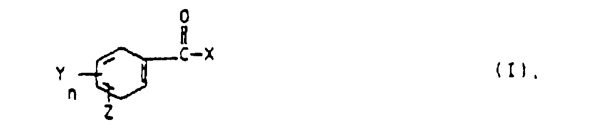 in which
X is an alkyl group containing 1 to 3 carbon atoms or is a phenyl radical optionally substituted by n groups Y,
Y is -H, -CF₃, -O-alkyl- and/or alkyl-COO- with in each case 1 to 4 carbon atoms in the alkyl group, halogen, -CN, -COOH or an -OH group which is not in ortho position,
n is 0 to 4 and
Z is a group of the general formula 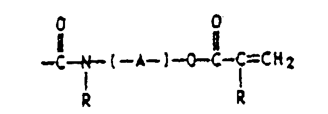 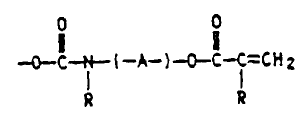 or 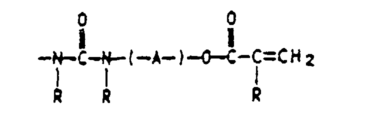 in which
R is H or C₁- to C₄-alkyl and
A is an alkylene, oxaalkylene or polyoxaalkylene radical having 2 to 12 carbon atoms,
c) the resulting solid polymer is heated and is applied in a form which is capable of flowing or a liquid form to a substrate, it being possible for the compositions of steps a) to c) to contain additives and auxiliaries if appropriate, and
d) the coated substrate is irradiated with high-energy and ionising radiation.

2. Process according to Claim 1, characterised in that the acrylic monomer a) is an alkyl ester of acrylic acid or methacrylic acid, the alkyl radical having 1 to 18 C atoms.

3. Process according to Claim 1, characterised in that the ethylenically unsaturated compounds contain polar groups.

4. Process according to Claim 1, characterised in that the content of ethylenically unsaturated compounds is 1 to 15% by weight, based on the total amount of monomers contained in the mixture.

5. Process according to Claim 1, characterised in that the copolymer is applied to a substrate at temperatures of 60 to 200°C.

6. Process according to Claim 1, characterised in that the dose of the high-energy radiation is 5 to 250 kGy.

7. Use of materials in the form of sheets or webs with an applied coating of a hot-melt pressure-sensitive adhesive obtainable by the process according to Claim 1 for the preparation of adhesive materials in the medical sector.

## Revendications

1. Procédé de préparation d'adhésifs à fusion à base d'acrylates par une irradiation sur un substrat, caractérisé
a) en ce que l'on copolymérise de 55 à 98 % en poids d'au moins un monomère acrylique avec de 2 à 30 % en poids de N-tert-butylacrylamide (TBA),
b) le mélange de réaction à polymériser (de a) pouvant en plus contenir un ou plusieurs composés éthyléniquement insaturés, qui n'appartiennent pas au groupe des monomères acryliques de a), et le mélange de réaction à polymériser (de a) ne contenant pas de masses réticulables aux ultraviolets à base de copolymérisats d'ester (méth)acrylique ayant une valeur K comprise entre 15 et 100, ayant une teneur comprise entre 0,01 et 10% en poids sur la base des copolymérisats, en monomères monopolymérisés de formule générale 1 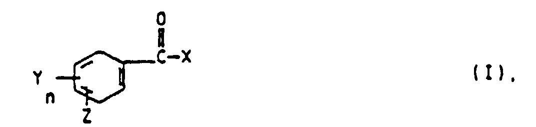 dans laquelle
X représente un groupe alkyle contenant de 1 à 3 atomes de carbone ou un résidu phényl éventuellement substitué par n groupes Y,
Y représente -H, -CF₃, -O-Alkyl- et/ou Alkyl-COO- comprenant de 1 à 4 atomes de C dans le groupe alkyle, halogéno, -CN, -COOH ou un groupement -OH ne se trouvant pas en position ortho,
n vaut de 0 à 4 et
Z représente un groupe de formule générale 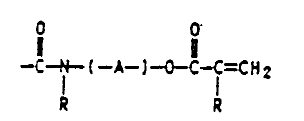 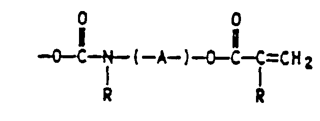 ou 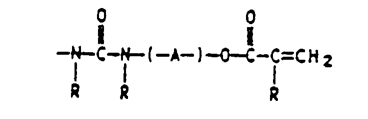 dans laquelle
R, H ou un alkyle en C₁ à C₂ et
A représente un radicale alkylène, Oxaalkylène ou polyoxaalkylène ayant de 2 à 12 atomes de carbone,
c) en ce que l'on chauffe le polymérisat solide obtenu et en ce que l'on procède à son application sous forme coulante ou sous forme fluide sur un substrat, les masses des étapes a) à c) pouvant, le cas échéant, contenir des additifs et des adjuvants, et
d) en ce que l'on procède à l'irradiation du substrat revêtu à l'aide d'un rayonnement riche en énergie et ionisant.

2. Procédé selon la revendication 1, caractérisé en ce que le monomère acrylique a) est un ester alkyle de l'acide acrylique ou de l'acide méthacrylique, le résidu alkyle possédant de 1 à 18 atomes de C.

3. Procédé selon la revendication 1, caractérisé en ce que les composés éthyléniquement insaturés contiennent des groupements polaires.

4. Procédé selon la revendication 1, caractérisé en ce que la proportion des composés éthyléniquement insaturés est de 1 à 15 % en poids, par rapport à la quantité totale des monomères contenus dans le mélange.

5. Procédé selon la revendication 1, caractérisé en ce que le polymérisat mixte est appliqué sur un substrat à des températures de 60 à 200°C.

6. Procédé selon la revendication 1, caractérisé en ce que la dose de rayonnement riche en énergie est de 5 à 250 kGy.

7. Utilisation de matériaux sous forme de feuilles ou sous forme de bandes ayant un enduit d'un adhésif à fusion, qui peut s'obtenir par un procédé selon la revendication 1, pour la préparation de matériaux adhésifs dans le domaine médical.
